# EUROPÄISCHE PATENTSCHRIFT

(11) **EP 2 932 962 B1**
(45) Veröffentlichungstag und Bekanntmachung des Hinweises auf die Patenterteilung: **13.10.2021**
(21) Anmeldenummer: 15154497.0
(22) Anmeldetag: 06.11.2007
(51) Int. Cl.: A61K 9/14, A61K 9/16, A61P 11/00, A61P 11/06, A61K 31/5375

(54) **Stabile Pulverformulierung enthaltend ein Anticholinergikum**
Stable powder formulation containing an anticholinergic agent
Formulation poudreuse stable contenant un nouvel anticholinergique

(30) Priorität: 22.11.2006 EP 06124580
(43) Veröffentlichungstag der Anmeldung: 21.10.2015
(62) Teilanmeldung aus: 07847111.7
(73) Patentinhaber: Boehringer Ingelheim International GmbH, 55216 Ingelheim am Rhein (DE); Boehringer Ingelheim Pharma GmbH & Co. KG, 55216 Ingelheim (DE)
(72) Erfinder: Trunk, Michael, 55216 INGELHEIM AM RHEIN (DE); Weiler, Claudius, 55216 INGELHEIM AM RHEIN (DE); Pieroth, Werner, 55216 INGELHEIM AM RHEIN (DE)
(74) Vertreter: Simon, Elke Anna Maria

(56) Entgegenhaltungen:
- EP-A1- 0 606 486
- WO-A-02/30928
- DE-A1- 4 425 255
- DE-A1- 10 331 350
- None

## Beschreibung

Die vorliegende Erfindung betrifft ein Verfahren zur Herstellung einer Pulverformulierung, die Partikel enthält, dadurch gekennzeichnet, dass
a) eine Lösung umfassend die Komponenten i) bis iii):
   i) Anticholinergikum der Formel **1** 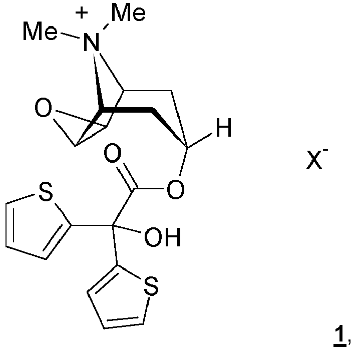 worin
      X ⁻ ein negativ geladenes Anion bedeutet,
   ii) mindestens ein Einbettungsmaterial ausgewählt aus der Gruppe bestehend aus Mono- oder Disacchariden, Oligosacchariden, Polymeren, Zuckeralkoholen und Cholesterin,
   iii) so viel einer organischen, physiologisch unbedenklichen Säure, dass die Lösung vor dem Zerstäuben gemäß Schritt b) einen pH von <7 besitzt, in einem geeigneten Lösungsmittel bereitgestellt wird, und
      nachfolgend die folgenden Schritte durchgeführt werden,
b) Zerstäuben der Lösung aus Schritt a) in einem Heißluftstrom
c) Trocknen der zerstäubten Lösung aus Schritt b) zu einem Pulver enthaltend Partikel.
d) Abscheidung des Pulvers aus Schritt c) mittels Zyklon oder Filters.

Verbindungen der Formel **1** sind aus der WO 02/30928 bereits bekannt. Sie besitzen wertvolle pharmakologische Eigenschaften und können als hochwirksame Anticholinergika bei der Therapie von Atemwegserkrankungen, vorzugsweise bei der Therapie entzündlicher und/oder obstruktiver Atemwegserkrankungen, insbesondere bei der Therapie von Asthma oder COPD (chronic obstructive pulmonary disease = chronisch obstruktive Lungenerkrankung) einen therapeutischen Nutzen entfalten. Die WO 02/30928 stellt weiterhin Arzneimittel in Form eines Inhalationspulvers mit einer Verbindung der Formel **1** als Wirkstoff beschrieben.

In der DE10331350A werden Pulverformulierungen für die Inhalation dargestellt, die Verbindungen nach Formel **1** zusammen mit einem physiologisch unbedenklichen Hilfsstoff ausgewählt aus der Gruppe der Monosaccharide, Disaccharide, Oligo- und Polysaccharide, Polyalkohole, Cyclodextrine und Aminosäuren enthalten, sowie Verfahren zur Herstellung dieser Pulverformulierungen. Die DE4425255A1 beschreibt Inhalationspulverzubereitungen, die unter anderem Ipratropiumbromid als Wirkstoff enthalten, wobei die Inhalationspulverzubereitungen durch geeignetes Mischen des Wirkstoffs oder des Wirkstoffgemisches mit einem pharmazeutisch verwendbaren Träger entsteht.

In der EP0606486A1 offenbart Pulverformulierungen zur Inhalation, die unter anderem Ipratropiumbromid als Wirkstoff und mindestens einen Hilfsstoff ausgewählt aus der Gruppe bestehend aus Hydroxypropylmethylcellulose, Methylcellulose, Hydroxypropylcellulose, Stärke, Hydroxyprooylstärke und Natriumalginat enthalten, wobei Sprühtrocknung als Möglichkeit zur Herstellung von Pulverformulierungen für Inhalativa erwähnt wird.

Keine der obengenannten Druckschriften stellt ein Verfahren zur Herstellung von Pulverformulierungen vor, in der so viel einer organischen, physiologisch unbedenklichen Säure zugegeben wird, dass die Lösung vor dem Zerstäuben gemäß Schritt b) einen pH von <7 besitzt.

Für das erfindungsgemäße Verfahren kann jede gängige Sprühtrocknungsvorrichtung verwendet werden, bei dem die Schritte a) bis d) durchgeführt werden.

In Schritt a) wird eine Lösung aus den Komponenten i) bis iii) in einem geeigneten Lösungsmittel bereitgestellt.

Vorzugsweise bedeutet X ⁻ in der Verbindung der Formel **1** ein Anion ausgewählt aus der Gruppe bestehend aus Chlorid, Bromid, lodid, Sulfat, Phosphat, Methansulfonat, Nitrat, Maleat, Acetat, Citrat, Fumarat, Tartrat, Oxalat, Succinat, Benzoat und p-Toluolsulfonat, insbesondere Bromid.

Die Lösung aus Schritt a) enthält hierbei als organische, physiologisch unbedenkliche, Säure nach iii) vorzugsweise eine Säure ausgewählt aus der Gruppe bestehend aus Ascorbinsäure, einer ein-, zwei- oder dreiwertigen Carbonsäure mit Ausnahme der Aminocarbonsäuren, und einer Frucht- oder Genusssäure.

Unter einer ein-, zwei- oder dreiwertigen Carbonsäure im Sinne der Erfindung werden C₂- bis C₁₀-, vorzugsweise C₃-bis C₆-Carbonsäuren mit jeweils einer, zwei oder drei Carboxylgruppen mit Ausnahme der Aminocarbonsäuren verstanden, wobei Fumarsäure, Oxalsäure, Bernsteinsäure, Citronensäure, Weinsäure, Äpfelsäure, Milchsäure, Essigsäure, a-Hydroxycaprylsäure oder Gluconsäure bevorzugt, Citronensäure besonders bevorzugt ist.

Unter einer Genuss- oder Fruchtsäure im Sinne der Erfindung werden vorzugsweise die Säuren ausgewählt aus der Gruppe Citronensäure, Weinsäure, Äpfelsäure, Milchsäure, Essigsäure, a-Hydroxycaprylsäure oder Gluconsäure, besonders bevorzugt Citronensäure, verstanden.

In einer weiteren bevorzugten Ausführungsform des erfindungsgemäßen Verfahrens wird der Lösung aus Schritt a), die bereits das Anticholinergikum nach i), vorzugsweise eine Verbindung nach Formel **1**, und mindestens ein Einbettungsmaterial nach ii) enthält, so viel der organischen, physiologisch unbedenklichen, sterisch anspruchsvollen Säure nach iii) zugegeben, dass die Lösung vor dem Zerstäuben gemäß Schritt b) einen pH von < 7, vorzugsweise von ≤ 6, stärker bevorzugt von ≤ 5, insbesondere von ≤ 4 besitzt.

In einer besonders bevorzugten Ausführungsform des erfindungsgemäßen Verfahrens wird der Lösung aus Schritt a), die bereits das Anticholinergikum nach i), vorzugsweise eine Verbindung nach Formel **1**, und mindestens ein Einbettungsmaterial nach ii) enthält, so viel der Citronensäure zugegeben, dass die Lösung vor dem Zerstäuben gemäß Schritt b) einen pH von < 7, vorzugsweise von ≤ 6, stärker bevorzugt von ≤ 5, insbesondere von ≤ 4 besitzt.

In einer weiteren besonders bevorzugten Ausführungsform enthält die Lösung aus Schritt a) zusätzlich zu den Komponenten i) bis iii) zusätzlich das Salz einer organischen, physiologisch unbedenklichen, Säure nach iii), die vorzugsweise ausgewählt ist aus der Gruppe bestehend aus Ascorbat, dem Salz einer ein-, zwei- oder dreiwertigen Carbonsäure mit Ausnahme der Salze der Aminocarbonsäuren, vorzugsweise Fumarat, Oxalat oder Succinat, und dem Salz einer Frucht- oder Genusssäure, vorzugsweise Citrat, Tartrat, Malat, Lactat, Acetat, α-Hydroxycapronat oder Gluconat. Besonders bevorzugt ist hierbei Citrat. Dieses zusätzliche Salz einer organischen, physiologisch unbedenklichenSäure nach iii) ist hierbei vorzugsweise ein Alkalimetallsalz, ein Erdalkalimetallsalz oder ein Zinksalz, vorzugsweise ein Alkalimetallcitrat, ein Erdalkalimetallcitrat oder ein Zinkcitrat, besonders bevorzugt Natriumcitrat, Kaliumcitrat, Magnesiumcitrat, Calciumcitrat und Zinkcitrat.

Vorzugsweise wird das Verhältnis der organischen, physiologisch unbedenklichen Säure, vorzugsweise Citronensäure, zum Salz der organischen, physiologisch unbedenklichen Säure, vorzugsweise Citrat, in der Lösung derart eingestellt, dass die in Schritt a) hergestellte Lösung enthaltend ein Anticholinergikum nach i), vorzugsweise eine Verbindung nach Formel **1** und mindestens ein Einbettungsmaterial nach ii) einen pH von < 7, vorzugsweise von ≤ 6, insbesondere von ≤ 5, stärker bevorzugt von ≤ 4 besitzt.

Vorzugsweise wird in Schritt a) eine solche Konzentration des Salzes der organischen, physiologisch unbedenklichen Säure eingesetzt, dass sich ein molares Verhältnis des Anticholinergikums nach i), insbesondere der Verbindung nach Formel **1**, zum Kation des Salzes von 1:1 bis 1:12, vorzugsweise von 1:2 bis 1:10 und insbesondere von 1:3 bis 1:8 ergibt.

Vorzugsweise wird als Einbettungsmaterial ii) ein Mono- oder Disaccharid ausgewählt aus der Gruppe bestehend aus Glucose, Saccharose, Fructose, Maltose, Lactose, Cellobiose und Trehalose, ein Oligosaccharid ausgewählt aus der Gruppe Oligomaltose, Oligofructose, Cyclodextrine, Dextrine und Oligosaccharose, ein Polymer ausgewählt aus der Gruppe Inulin, Alginat, Maltodextrin, Stärke, Stärkederivate, Cellulose, Cellulosederivate, PVP (Plasdone), Gelatine, Chitosan, Dextrane, Pektine, Gummi Arabicum, Polylaktide, Poly(laktid-co-glykolide) und Polyvinylalkohole, ein Zuckeralkohol ausgewählt aus der Gruppe Mannitol, Xylitol und Sorbitol oder Cholesterin eingesetzt. Besonders bevorzugt als Einbettungsmaterial ii) sind jedoch Lactose, Trehalose und Mannitol, insbesondere Lactose.

Vorzugsweise wird die Verbindung der Formel **1** nach i) in einer Konzentration von 0,04g/100ml bis 0,4g/100ml, das Einbettungsmaterial nach ii) in einer Konzentration von 5g/100ml bis 15g/100ml im Lösungsmittel gelöst und die organische, physiologisch unbedenkliche, sterisch anspruchsvolle Säure nach iii) in einer solchen Konzentration zugegeben, dass ein pH von < 7, vorzugsweise von ≤ 6, stärker bevorzugt von ≤ 5, insbesondere von ≤ 4 eingestellt wird.

Als Lösungsmittel kann Wasser, jedes geeignete organische Lösungmittel, ein Wasser/ organisches Lösungsmittel-Gemisch und ein Gemisch aus verschiedenen organischen Lösungsmitteln verwendet werden. Bevorzugt ist die Verwendung von Wasser, Ethanol und einem Ethanol/Wasser-Gemisch als Lösungsmittel.

In einer besonders bevorzugten Ausführungsform des erfindungsgemäßen Verfahrens erfolgt das Zerstäuben der Lösung aus Schritt a) in Schritt b) mittels einer Düse. Diese Düse wird vorzugsweise durch Flüssigkeitsdruck oder Pressluft bzw. Inertgas betrieben. Der Zerstäubungsdruck wird derart gewählt, dass Tropfengrößen im Bereich von <20 µm entstehen. Die Tröpfchengrößen der zerstäubten Lösung können beispielsweise mit Hilfe eines Laserbeugungsverfahrens mit einem Laser der Firma Sympatec (50 mm Brennweite, Mie-Auswertung) experimentell bestimmt werden. Vorzugsweise besitzt der Heißluftstrom aus Schritt b) Temperaturen zwischen 100 und 350°C, insbesondere zwischen 120 und 200°C.

In einer weiteren bevorzugten Ausführungsform des erfindungsgemäßen Verfahrens entstehen nach dem Trocknen in Schritt c) sprühgetrocknete Partikel, die einen mittleren aerodynamischen Durchmesser von ≤ 15 µm, vorzugsweise von ≤ 10 µm, insbesondere von ≤ 5 µm besitzen. Besonders bevorzugt ist ein erfindungsgemäßes Verfahren, bei dem nach dem Trocknen in Schritt c) sprühgetrocknete Partikel entstehen, die "inhalierfähig" sind, d.h. die einen Durchmesser haben, der klein genug ist, um eine topische Applikation in der Lunge zu bewirken.

Die Erfindung betrifft weiterhin eine sprühgetrocknete Pulverformulierung enthaltend Partikel, die durch eines der oben genannten erfindungsgemäßen Verfahren erhältlich ist.

### BEISPIELE

### Formulierungsbeispiele:

### Lösungen nach Schritt a) mit Citronensäure:

**Lösung vor der Sprühtrocknung:**

| | |
|---|---|
| Verbindung nach Formel **1** | 0,4 g (1% bez. auf den Feststoff) |
| (X ⁻ = BR ⁻) | |
| Lactose | 40,6 g |
| Citronensäure | 0,25 g (Zugabe bis pH 3; 0,6% bezogen auf den Gesamt-Feststoff der Partikel) |
| Wasser | 460 g |

**Lösungen nach Schritt a) mit Citronensäure und Citrat:**

| | |
|---|---|
| Lösung vor der Sprühtrocknung: | |
| Verbindung nach Formel **1** | 0,4 g (1% bez. auf den Feststoff) |
| (X ⁻ = BR ⁻) | |
| Lactose | 38,5 g |
| Citronensäure | 2,1 g (Zugabe bis pH 3; 5,2% bezogen auf den Gesamt-Feststoff der Partikel) |
| Zinkcitrat Dihydrat | 1,0 g (2,5% bezogen auf den Gesamt-Feststoff der Partikel) molares Verhältnis des Anticholinergikums zum Zink-Ion beträgt 1:6 |
| Wasser | 460 g |

### Herstellungsbeispiele:

### Sprühgetrocknete Pulverformulierung (mit Citronensäure):

### Beispiel 1: Sprühparameter, geeignet zur Erzeugung inhalierbarer Partikel mit Ba 679

### Durchführung:

In einem Erlenmeyerkolben wird das Lösungsmittel vorgelegt. Die Zugabe des Einbettungsmaterial erfolgt portionsweise unter starkem Rühren (z.B. Magnetrührer) und ggf. unter Erwärmung. Sobald die Lösung klar ist, erfolgt die Einstellung des pH-Wertes mit Citronensäure auf pH = 3 und die Zugabe der Verbindung nach Formel **1**. Nach vollständigem Lösen schließt sich umgehend die Sprühtrocknung an.

Die Sprühtrocknung erfolgt mittels einem modifizierten BÜCHI Mini-Spray Dryer (B-191) in Verbindung mit einer modifizierten 0,5 mm Zwei-Stoff-Düse und unter der ausschließlichen Verwendung von N₂ als Prozess- und Düsengas. Im Wesentlichen wurden hierbei alle Glasteile durch Metallteile ersetzt und der Aspirator entfernt. Über den Prozessgaseintritt wird N₂ als Trockengas zugeführt (ca. 35 m³/h) so dass das Gerät im Überdruckbereich durchströmt wird. Der Ausgangsfilter zwischen Zyklon und Aspirator wurde entfernt und der Gasaustritt direkt nach dem Zyklon in einen Abzug mit integriertem Partikelfeinfilter geleitet. Die Zweistoffdüse wurde aus Edelstahl gefertigt, wobei die 0,5 mm Düsenkappe mit Mischnadel und Düsenüberwurfmutter als zentrale Zerstäubungseinheit beibehalten wurden. Der Massenstrom des Düsengasdurchsatzes wird über ein externes Messgerät (Kobold MAS 3015) ermittelt und von dem ursprünglichen Schwebekörperdurchflussmesser entkoppelt. Üblicherweise wird die Düse bei einem Gasdruck von ca. 6 bar Überdruck betrieben. Die Eingangstemperatur des Prozessgases beträgt 150°C. Der Massenstrom der Sprühlösung ist derart zu wählen, dass eine Ausgangstemperatur von 82±3°C gegeben ist. Nach erfolgter Sprühtrocknung ist das Pulver umgehend zu entnehmen und unter Ausschluss von Feuchtigkeit zu Lagern bzw. weiter zu verarbeiten. Die verwendeten Prozessparameter sind in Tabelle 1 ersichtlich.

**Tabelle 1. Sprühtrocknungsparameter**

| | |
|---|---|
| Volumenstrom "Sprührate" | 12 ml/min |
| Sprühdruck (Düsentyp) | 6 bar Überdruck N₂ (BÜCHI Sprühdüse 0.5 mm, modifiziert) |
| Volumenstrom "Zerstäubungsdruck" (Düsentyp) | 2580 Normliter / h (BÜCHI Sprühdüse 0.5 mm, modifiziert) |
| Eingangstemperatur | 150°C |
| Ausgangstemperatur | 80°C |
| Volumenstrom "Trocknungsgas" | 31 Norm m³ / h |
| Querschnitt Trockenturm | 105 mm |

### Sprühgetrocknete Pulverformulierung (mit Citronensäure und Citrat):

### Beispiel 2: Sprühparameter, geeignet zur Erzeugung inhalierbarer Partikel mit Ba 679

### Durchführung:

In einem Erlenmeyerkolben wird das Lösungsmittel vorgelegt. Die Zugabe des Einbettungsmaterial erfolgt portionsweise unter starkem Rühren (z.B. Magnetrührer) und ggf. unter Erwärmung. Sobald die Lösung klar ist, erfolgt die Zugabe des Salzes einer organischen Säure und die Einstellung des pH-Wertes mit Citronensäure auf pH = 3. Anschließend erfolgt die Zugabe der Verbindung nach Formel **1**. Nach vollständigem Lösen schließt sich umgehend die Sprühtrocknung an.

Die Sprühtrocknung erfolgt wie unter Beispiel 1 beschrieben. Die verwendeten Prozessparameter sind in Tabelle 2 ersichtlich.

**Tabelle 2. Sprühtrocknungsparameter**

| | |
|---|---|
| Volumenstrom "Sprührate" | 12 ml/min |
| Sprühdruck (Düsentyp) | 6 bar Überdruck N2 (BÜCHI Sprühdüse 0.5 mm, modifiziert) |
| Volumenstrom "Zerstäubungsdruck" (Düsentyp) | 2580 Normliter / h (BÜCHI Sprühdüse 0.5 mm, modifiziert) |
| Eingangstemperatur | 150°C |
| Ausgangstemperatur | 79°C |
| Volumenstrom "Trocknungsgas" | 31 Norm m3 / h |
| Querschnitt Trockenturm | 105 mm |

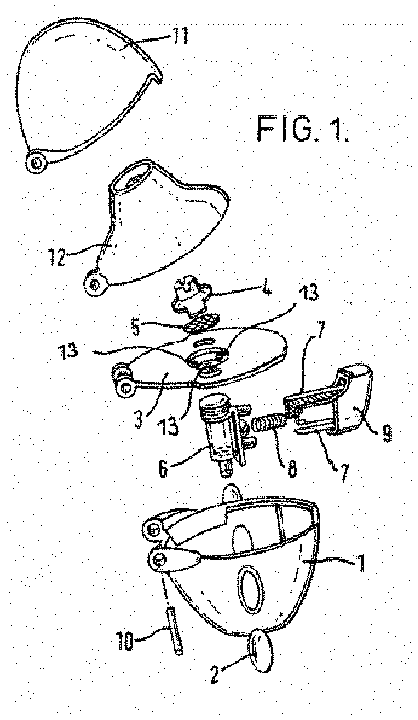

## Patentansprüche

1. Verfahren zur Herstellung einer Pulverformulierung, die Partikel enthält, **dadurch gekennzeichnet, dass**
a) eine Lösung umfassend die Komponenten i) bis iii):
i) Anticholinergikum der Formel **1** 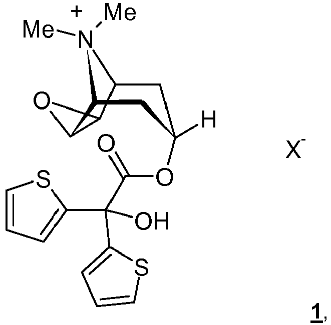 worin
X ⁻ ein negativ geladenes Anion bedeutet,
ii) mindestens ein Einbettungsmaterial ausgewählt aus der Gruppe bestehend aus Mono- oder Disacchariden, Oligosacchariden, Polymeren, Zuckeralkoholen und Cholesterin,
iii) so viel einer organischen, physiologisch unbedenklichen Säure, dass die Lösung vor dem Zerstäuben gemäß Schritt b) einen pH von <7 besitzt, in einem geeigneten Lösungsmittel bereitgestellt wird, und
nachfolgend die folgenden Schritte durchgeführt werden,
b) Zerstäuben der Lösung aus Schritt a) in einem Heißluftstrom
c) Trocknen der zerstäubten Lösung aus Schritt b) zu einem Pulver enthaltend Partikel.
d) Abscheidung des Pulvers aus Schritt c) mittels Zyklon oder Filters.

2. Verfahren nach Anspruch 1, wobei in Schritt a) eine Lösung bereitgestellt wird, die eine Verbindung der Formel **1** enthält, worin X ⁻ ein Anion ausgewählt aus der Gruppe bestehend aus Chlorid, Bromid, lodid, Sulfat, Phosphat, Methansulfonat, Nitrat, Maleat, Acetat, Citrat, Fumarat, Tartrat, Oxalat, Succinat, Benzoat und p-Toluolsulfonat bedeutet.

3. Verfahren nach einem der Ansprüche 1 oder 2, wobei in Schritt a) eine Lösung bereitgestellt wird, die eine Verbindung der Formel **1** umfasst, worin X ⁻Bromid ist.

4. Verfahren nach einem der Ansprüche 1 bis 3, wobei in Schritt a) die Lösung einen pH von ≤ 6, insbesondere von ≤ 5, stärker bevorzugt von ≤ 4 besitzt.

5. Verfahren nach einem der Ansprüche 1 bis 4, wobei das Einbettungsmaterial der Lösung aus Schritt a) ein Mono- oder Disaccharid ausgewählt aus der Gruppe bestehend aus Glucose, Saccharose, Fructose, Maltose, Lactose, Cellobiose und Trehalose ist oder ein Oligosaccharide ausgewählt aus der Gruppe bestehend aus Oligomaltose, Oligofructose, Cyclodextrine, Dextrine und Oligosaccharose ist oder ein Polymer ausgewählt aus der Gruppe bestehend aus Inulin, Alginat, Maltodextrin, Stärke, Stärkederivate, Cellulose, Cellulosederivate, PVP, Gelatine, Chitosan, Dextrane, Pektine, Gummi Arabicum, Polylaktide, Poly(laktid-co-glykolide) und Polyvinylalkohole ist oder ein Zuckeralkohol ausgewählt aus der Gruppe bestehend aus Mannitol, Xylitol und Sorbitol oder Cholesterin ist.

6. Verfahren nach einem der Ansprüche 1 bis 5, wobei die Partikel aus Schritt c) einen mittleren Durchmesser von maximal 15 µm, vorzugsweise von maximal 10 µm besitzen.

7. Verfahren nach einem der Ansprüche 1 bis 6, wobei das Zerstäuben nach Schritt b) mittels einer Düse erfolgt.

8. Verfahren nach einem der Ansprüche 1 bis 7, wobei der Heißluftstrom nach Schritt b) Temperaturen zwischen 100 und 350°C, vorzugsweise zwischen 120 und 200 °C besitzt.

9. Pulverformulierung erhältlich durch ein Verfahren nach einem der Ansprüche 1 bis 8.

10. Pulverformulierung nach Anspruch 9, welche weitere physiologisch unbedenkliche Hilfsstoffe enthält.

## Claims

1. Process for producing a powder formulation which contains particles, **characterized in that**
a) a solution comprising components i) to iii):
i) an anticholinergic of formula **1** 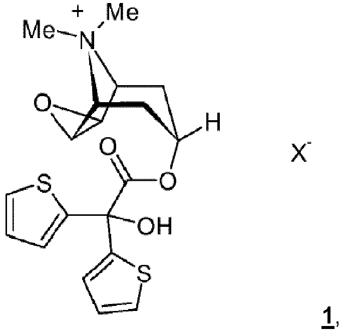 wherein
X⁻ denotes a negatively charged anion,
ii) at least one embedding material selected from the group consisting of mono- or disaccharides, oligosaccharides, polymers, sugar alcohols and cholesterol,
iii) a sufficient amount of an organic, physiologically acceptable acid such that the solution has a pH of <7 before atomization according to step b), is provided in a suitable solvent, and
subsequently the following steps are carried out,
b) atomizing the solution from step a) in a hot air current
c) drying the atomized solution from step b) to form a powder containing particles
d) separating off the powder from step c) using a cyclone or filters.

2. Process according to claim 1, wherein in step a) a solution is provided which contains a compound of formula **1,** wherein X⁻ denotes an anion selected from the group consisting of chloride, bromide, iodide, sulphate, phosphate, methanesulphonate, nitrate, maleate, acetate, citrate, fumarate, tartrate, oxalate, succinate, benzoate and p-toluenesulphonate.

3. Process according to one of claims 1 or 2, wherein in step a) a solution is provided which comprises a compound of formula **1,** wherein X⁻ is bromide.

4. Process according to one of claims 1 to 3, wherein in step a) the solution has a pH of ≤6, in particular ≤5, more preferably ≤4.

5. Process according to one of claims 1 to 4, wherein the embedding material of the solution from step a) is a mono- or disaccharide selected from the group consisting of glucose, saccharose, fructose, maltose, lactose, cellobiose and trehalose or an oligosaccharide selected from the group consisting of oligomaltose, oligofructose, cyclodextrins, dextrins and oligosaccharose or a polymer selected from the group consisting of inulin, alginate, maltodextrin, starch, starch derivatives, cellulose, cellulose derivatives, PVP, gelatin, chitosan, dextrans, pectins, gum arabic, polylactides, poly(lactide-co-glycolides) and polyvinylalcohols or a sugar alcohol selected from the group consisting of mannitol, xylitol and sorbitol or cholesterol.

6. Process according to one of claims 1 to 5, wherein the particles from step c) have a median diameter of at most 15 µm, preferably of at most 10 µm.

7. Process according to one of claims 1 to 6, wherein the atomization according to step b) takes place using a nozzle.

8. Process according to one of claims 1 to 7, wherein the hot air current according to step b) is at temperatures between 100 and 350°C, preferably between 120 and 200°C.

9. Powder formulation which may be obtained by a process according to one of claims 1 to 8.

10. Powder formulation according to claim 9, which contains further physiologically acceptable auxiliaries.

## Revendications

1. Procédé de fabrication d'une formulation de poudre, qui contient des particules, **caractérisé en ce que**
a) est fournie dans un solvant adapté une solution comprenant les composants i) à iii) :
i) un agent anticholinergique de la formule 1 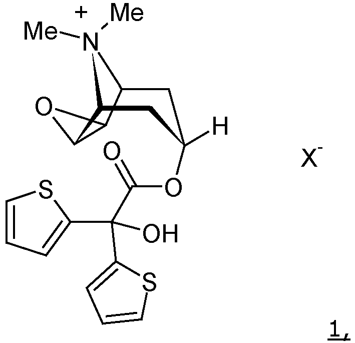 dans laquelle
X⁻ signifie un anion chargé négativement,
ii) au moins un matériau d'enrobage choisi parmi le groupe constitué de mono- ou de disaccharides, d'oligosaccharides, de polymères, d'alcools de sucre et de cholestérine ;
iii) une quantité si importante d'un acide organique physiologiquement acceptable que la solution possède avant la pulvérisation selon l'étape b) un pH < 7, et
les étapes suivantes sont ensuite effectuées
b) la pulvérisation de la solution issue de l'étape a) dans un flux d'air chaud,
c) le séchage de la solution pulvérisée issue de l'étape b) en une poudre contenant des particules,
d) la séparation de la poudre issue de l'étape c) au moyen d'un cyclone ou d'un filtre.

2. Procédé selon la revendication 1, dans lequel est fournie à l'étape a) une solution qui contient un composé de la formule 1, dans laquelle X⁻ signifie un anion choisi parmi le groupe constitué de chlorure, de bromure, d'iodure, de sulfate, de phosphate, de sulfonate de méthane, de nitrate, de maléate, d'acétate, de citrate, de fumarate, de tartrate, d'oxalate, de succinate, de benzoate et de p-toluènesulfonate.

3. Procédé selon l'une quelconque des revendications 1 ou 2, dans lequel est fournie à l'étape a) une solution qui comprend un composé de la formule 1, dans laquelle X⁻ est du bromure.

4. Procédé selon l'une quelconque des revendications 1 à 3, dans lequel la solution possède, à l'étape a), un pH ≤ 6, en particulier ≤ 5, de manière davantage préférée ≤ 4.

5. Procédé selon l'une quelconque des revendications 1 à 4, dans lequel le matériau d'enrobage de la solution issue de l'étape a) est un mono- ou disaccharide choisi parmi le groupe constitué de glucose, de saccharose, de fructose, de maltose, de lactose, de cellobiose et de tréhalose ou est un oligosaccharide choisi parmi le groupe constitué d'oligomaltose, d'oligofructose, de cyclodextrine, de dextrine et d'oligosaccharose ou est un polymère choisi parmi le groupe constitué d'inuline, d'alginate, de maltodextrine, d'amidon, de dérivés d'amidon, de cellulose, de dérivés de cellulose, de PVP, de gélatine, de chitosane, de dextrane, de pectine, de gomme arabique, de polyactide, de poly(lactide-co-glycolide) et d'alcools polyvinyliques ou un alcool de sucre choisi parmi le groupe constitué de mannitol, de xylitol et de sorbitol ou de cholestérine.

6. Procédé selon l'une quelconque des revendications 1 à 5, dans lequel les particules issues de l'étape c) possèdent un diamètre moyen de 15 µm au maximum, de préférence de 10 µm au maximum.

7. Procédé selon l'une quelconque des revendications 1 à 6, dans lequel la pulvérisation après l'étape b) est effectuée au moyen d'une buse.

8. Procédé selon l'une quelconque des revendications 1 à 7, dans lequel le flux d'air chaud possède après l'étape b) des températures entre 100 et 350 °C, de préférence entre 120 et 200 °C.

9. Formulation de poudre pouvant être obtenue par un procédé selon l'une quelconque des revendications 1 à 8.

10. Formulation de poudre selon la revendication 9, laquelle contient des excipients supplémentaires physiologiquement acceptables.
